# EUROPÄISCHE PATENTSCHRIFT

(11) **EP 2 547 713 B1**
(45) Veröffentlichungstag und Bekanntmachung des Hinweises auf die Patenterteilung: **23.04.2014**
(21) Anmeldenummer: 11706848.6
(22) Anmeldetag: 09.03.2011
(51) Int. Cl.: C08G 65/00

(54) **Verfahren zur Herstellung von Polyetherpolyolen**
Method for manufacturing polyether polyols
Procédé destiné à la fabrication de polyols de polyéther

(30) Priorität: 13.03.2010 EP 10002671
(43) Veröffentlichungstag der Anmeldung: 23.01.2013
(73) Patentinhaber: Bayer Intellectual Property GmbH, 40789 Monheim (DE)
(72) Erfinder: LORENZ, Klaus, 41539 Dormagen (DE)
(74) Vertreter: BIP Patents
(86) Internationale Anmeldenummer: PCT/EP2011/053501
(87) Internationale Veröffentlichungsnummer: WO 2011/113729

(56) Entgegenhaltungen:
- EP-A1- 0 037 010
- EP-A2- 0 618 251
- WO-A2-2007/096367
- DE-A1- 4 209 358

## Beschreibung

Die vorliegende Erfindung betrifft ein Koppelverfahren zur Herstellung von Polyetherpolyolen ausgehend von hochfunktionellen, bei Reaktionsbedingungen festen bzw. hochviskosen Starterverbindungen und monofunktionellen, bei Reaktionsbedingungen flüssigen Starterverbindungen mit Zerewitinoff-aktiven Wasserstoffatomen. Unter einer hochfunktionellen Starterverbindung im Sinne der Erfindung werden Verbindungen bezeichnet, die mindestens vier Zerewitinoff-aktive Wasserstoffatome (d.h. beispielsweise über Hydroxy- und/oder Aminfunktionalität) aufweisen.

Polyole auf Basis hochfunktioneller Starterverbindungen wie beispielsweise Zucker, Oligo- und Polysaccharide, Zuckeralkohole (wie beispielsweise Mannit oder Sorbit) sowie Pentaerythrit werden in der Regel in Polyurethananwendungen eingesetzt, insbesondere zur Herstellung von Polyurethanhartschaumstoffen, die für die Isolierung von Kältegeräten, Kühlcontainern oder auch bei der Gebäudeisolierung eingesetzt werden. Polyolformulierungen basierend auf solchen, auf Basis hochfunktioneller Starterverbindungen hergestellten Polyolen, können mit Polyisocyanaten zu Hartschaumstoffen verarbeitet werden, wobei daraus hergestellte Isolierelemente (wie beispielsweise Kühlschrankgehäuse oder Isolierpaneele) schnell aus den Schäumformen entnommen werden können, ohne dass nennenswerte Verformungen der Isolierelemente eintreten.

Zucker (Saccharose), andere Oligo- und Polysaccharide bzw. Kondensationsprodukte wie Pentaerythrit, Dipentaerythrit, Tripentaerythrit und Trimethylolethan weisen in der Regel Schmelzpunkte auf, die nahe oder oberhalb der Reaktionstemperatur für die Herstellung von Polyetherpolyolen durch Alkylenoxidaddition liegen oder sie zersetzen sich vor Erreichen der Schmelztemperatur. Der Stand der Technik kennt daher viele Verfahren, diese Starterverbindungen Alkylenoxidadditionsreaktionen zugänglich zu machen. Beispielsweise können, wie in US 4,332,936 beschrieben, zur Suspendierung der festen Starter Lösungsmittel eingesetzt werden. Als nachteilig erweist sich hier, dass wertvolles Reaktorvolumen für das Lösungsmittel zur Verfügung gestellt werden muss und aus Gründen der Nachhaltigkeit und Produkthygiene die Verwendung von organischen Lösungsmitteln im Allgemeinen nicht erwünscht ist.

Des Weiteren besteht die Möglichkeit die bei Reaktionsbedingungen festen, hochviskosen bzw. sich unterhalb oder bei Reaktionstemperatur zersetzenden Starterverbindungen in Gegenwart anderer bei Reaktionsbedingungen flüssiger Starterverbindungen mit Alkylenoxiden umzusetzen. Solche Vorgehensweisen sind beschrieben in US 3153002, DE-A 2241242, DD-A 146606, DD-A 147469, DD-A 219204, DD-A 216248, DE-A 3011083, DE-A 4209358, DE-A 10237918 und DE-A 10237914. Diese Verfahren bedienen sich ausnahmslos des Mischstarts der bei Reaktionsbedingungen festen, hochviskosen bzw. sich unterhalb oder bei Reaktionstemperatur zersetzenden Starterverbindungen mit anderen, flüssigen, zur Suspendierung bzw. teilweisen Auflösung dieser Starter geeigneten Starterverbindungen mit Hydroxy- oder Aminfunktionalitäten von 2 bis 6. Man erhält zwangsläufig Endprodukte mit niedrigeren Funktionalitäten als die der reinen hochschmelzenden bzw. hochviskosen und / oder zersetzungsempfindlichen Starterverbindungen. Solche mischgestarteten Polyole sind daher zur Herstellung von modernen Hartschaumstoffformulierungen, die höchste Ansprüche an die Entformbarkeit der mit ihnen hergestellten Isolierelemente erfüllen müssen, weniger gut geeignet.

Als Suspensionsmittel können auch Alkylenoxidadditionsprodukte von mehrfunktionellen Starterverbindungen eingesetzt werden, wie beispielsweise in FR-A 1285708 und US 3,190,927 beschrieben. Werden hierbei die Endprodukte selbst oder Zwischenprodukte auf Basis der bei Reaktionsbedingungen festen, hochviskosen bzw. sich unterhalb oder bei Reaktionstemperatur zersetzenden Starterverbindungen verwendet, lassen sich im Prinzip ausschließlich auf Basis der hochschmelzenden, bzw. zersetzungsempfindlichen Starterverbindungen basierende Polyetherpolyole erhalten. Als nachteilig hat sich das unzureichende Lösungsvermögen der Endprodukte für die hochfunktionellen Starterverbindungen erwiesen, außerdem muss, ebenso wie beim Einsatz von Lösungsmitteln, wertvolles Reaktorvolumen für das Suspendierungsmittel geopfert werden.

Wird Wasser als Suspendierungs- / Lösungsmittel für die bei Reaktionsbedingungen festen, hochviskosen bzw. sich unterhalb oder bei Reaktionstemperatur zersetzenden Starterverbindungen eingesetzt, so kann die Alkylenoxidadditionsreaktion an geeigneter Stelle unterbrochen werden und das noch unreagierte Wasser abdestilliert werden. Solche Vorgehensweisen sind beschrieben in DE-A 1443022 und US 4,430,490. Diese sogenannten Wasserverfahren haben den Nachteil, dass das als Suspendierungs- und Lösungsmittel verwendete Wasser bei der Alkylenoxidadditionsreaktion in gewissem Ausmaß mitreagiert und die Funktionalität der Endprodukte damit zum Einen sinkt und auch weniger gut kontrollierbar ist als bei Verwendung von hydroxygruppen- oder amingruppenhaltigen flüssigen Costartern. Zum Anderen entstehen glykolhaltige Abwässer, die entweder aufgereinigt werden müssen oder deren Glykolgehalt bei Rückführung in den Prozess auf einen konstanten Wert eingestellt werden muss.

Ein Verfahren, mit dem rein auf Saccharosebasis gestartete Polyetherpolyole erhältlich sind, ist in US 2902478 beschrieben. Trimethylamin wird als Katalysator und das Monomer Propylenoxid als Suspendierungsmittel verwendet. Eine solche Reaktionsführung ist risikoreich und daher aus Sicherheitsgründen nicht empfehlenswert.

In US 4,385,173 und DD-A 200427 werden apparativ aufwändige Vorgehensweisen beschrieben mit Hilfe derer rein auf Basis der bei Reaktionsbedingungen festen, hochviskosen bzw. sich unterhalb oder bei Reaktionstemperatur zersetzenden Starterverbindungen gestartete Polyetherpolyole erhältlich sind. Die verwendeten Reaktortypen sind nicht universell einsetzbar, d. h. für Alkylenoxidadditionsreaktionen an anderen Starterverbindungen ungeeignet.

Aufgabe der vorliegenden Erfindung war es ein Verfahren zur Herstellung von möglichst hochfunktionellen Polyetherpolyolen auf Basis von hochfunktionellen Starterverbindungen zu finden, wobei sich die einzusetzenden hochfunktionellen Starterverbindungen nahe oder erst oberhalb der üblichen Reaktionstemperatur von Alkylenoxidadditionsverfahren von etwa 100 °C schmelzen, oder im Bereich der Reaktionstemperatur von etwa 100 °C eine zu hohe Viskosität besitzen, welches die geschilderten Nachteile der Verfahren des Standes der Technik nicht aufweist. Vorzugsweise soll das aus der hochfunktionellen Starterverbindung resultierende Polyetherpolyol in einer Form hergestellt werden, dass maximal 15 Gew.-% eines auf einer weiteren Starterverbindung resultierenden Polyetherpolyols enthalten ist.

Überraschenderweise wurde gefunden, dass diese Aufgabe gelöst wird durch ein Koppelverfahren zur Herstellung eines ersten Polyetherpolyols A und eines zweiten, monofunktionellen Polyetherpolyols B, dadurch gekennzeichnet, dass
(i) eine Lösung bzw. Dispersion einer oder mehrerer Starterverbindungen (S-1), die jeweils mindestens vier Zerewitinoff-aktive Wasserstoffatome aufweisen, in einer oder mehreren monofunktionellen Starterverbindungen (S-2) hergestellt wird, wobei das Gewichtsverhältnis von S-1 zu S-2 20 : 80 bis 85 : 15, bevorzugt 50 : 50 bis 85 : 15, besonders bevorzugt 60 : 40 bis 85 : 15 beträgt,
(ii) die aus Schritt (i) resultierende Lösung bzw. Dispersion mit einem oder mehreren Alkylenoxiden, gegebenenfalls nach Zusatz eines Katalysators, zur Reaktion gebracht wird, wobei das Umsetzungsprodukt aus der mindestens einen Starterverbindung (S-1) mit einem oder mehreren Alkylenoxiden das Polyetherpolyol A ist, und wobei das Umsetzungsprodukt aus der mindestens einen monofunktionellen Starterverbindung (S-2) mit einem oder mehreren Alkylenoxiden das monofunktionelle Polyetherpolyol B ist,
(iii) gegebenenfalls das resultierende Gemisch vom Katalysator befreit wird, und
(iv) das monofunktionelle Polyetherpolyol B durch Destillation unter vermindertem Druck oder durch Strippen mit Inertgas oder Wasser abgetrennt wird, wobei der verbleibende Rückstand Polyetherpolyol A und bis zu 15 Gew.-% (jeweils bezogen auf die Summe der Gew.-% des Polyetherpolyols A und des monofunktionellen Polyetherpolyols B) an monofunktionellem Polyetherpolyol B enthält.

Das erfindungsgemäße Verfahren hat den Vorteil, dass hochfunktionelle, bei Reaktionstemperatur noch feste oder zu viskose Starterverbindungen (S-1) zur Alkoxylierung eingesetzt werden können. Die aus der Alkoxylierung der im Verfahren ebenfalls eingesetzten monofunktionellen Starterverbindungen (S-2) entstehenden monofunktionellen Polyether lassen sich aus dem Reaktionsgemisch durch Destillation entfernen, so dass sich die aus der mindestens einen Starterverbindung (S-1) resultierenden hochfunktionellen Alkylenoxidadditionsprodukte in hoher Reinheit (d.h. mindestens 85 Gew.-% ig) isolieren lassen. Die hochfunktionellen Alkylenoxidadditionsprodukte können als Komponenten von Hartschaumstoffformulierungen mit Polyisocyanaten verarbeitet werden, wobei die resultierenden Isolierelemente höchste Ansprüche an die Entformbarkeit erfüllen. Ebenfalls können die hochfunktionellen Alkylenoxidadditionsprodukte weiteren Alkylenoxidadditionsreaktionen unterzogen werden, womit auf anderem Wege nicht leicht erhältliche hochfunktionelle Polyetherpolyole mit hohen Äquivalentmolmassen zugänglich werden. Die durch Destillation entfernten monofunktionellen Polyether finden ihrerseits Verwendung beispielsweise als wertvolle Lösungsmittel oder als Starterverbindungen für höhermolekulare monofunktionelle Polyether, die beispielsweise als Tenside oder als Schmiermittel eingesetzt werden können.

An N, O oder S gebundener Wasserstoff wird als Zerewitinoff-aktiver Wasserstoff (oder als "aktiver Wasserstoff") bezeichnet, wenn er nach einem von Zerewitinoff aufgefundenen Verfahren durch Umsetzung mit Methylmagnesiumjodid Methan liefert. Typische Beispiele für Verbindungen mit Zerewitinoff-aktivem Wasserstoff sind Verbindungen, die Carboxyl-, Hydroxyl-, Amino-, Imino- oder Thiol-Gruppen als funktionelle Gruppen enthalten.

Bei den Starterverbindungen (S-1) handelt es sich um mindestens eine Verbindung ausgewählt aus der Gruppe bestehend aus Mono-, Oligo- und Polysaccharide, Pentaerythrit, Dipentaerythrit, Tripentaerythrit, , Sorbit, cyclischen Polyolen (wie beispielsweise Inosit), Polyamine (wie beispielsweise Verbindungen auf Basis hochfunktioneller mehrkerniger Anilin / Formaldehyd - Kondensationsprodukte ("polymeres MDA")) und Isomere bzw. Isomerengemische des Toluylendiamins (insbesondere 2,4-TDA, 2,6-TDA, 2,3-TDA, 3,4-TDA). Bevorzugt wird als Starterverbindung (S-1) Saccharose und/oder Pentaerythrit eingesetzt, besonders bevorzugt wird Saccharose eingesetzt. Die Starterverbindungen (S-1) liegen im Allgemeinen bei 70 °C (dies ist üblicherweise die untere Grenze des Reaktionstemperaturbereiches bei Alkoxylierungen) in fester Form vor oder sie sind hochviskos (d.h. sie weisen bei 70°C eine Viskosität von 100 mPas oder mehr auf).

Bei den monofunktionellen Starterverbindungen (S-2) handelt es sich um mindestens eine Verbindung ausgewählt aus der Gruppe bestehend aus monofunktionellen Alkoholen, kurzkettigen Monoalkylethern von Glykolen (beispielsweise Ethylenglykolmonomethylether oder Diethylenglykolmonomethylether), höheren monofunktionellen Fettalkoholen (beispielsweise monofunktionelle Fettalkohole mit 8 bis 30 Kohlenstoffatomen wie beispielsweise 10-Undecen-1-ol oder 1-Decanol), monofunktionellen Aminen (beispielsweise Dimethylamin, Methylethylamin, Diethylamin) und monofunktionellen Alkanolaminen (wie beispielsweise 2-(Dimethylamino)-ethanol und 1-(Dimethylamino)-2-propanol). Bevorzugt handelt es sich bei den monofunktionellen Starterverbindungen (S-2) um mindestens eine Verbindung ausgewählt aus der Gruppe bestehend aus Methanol, Ethanol, Propanol (beispielsweise 1-Propanol, Isopropanol), Butanol (beispielsweise 1-Butanol, 2-Butanol), Pentanol (beispielsweise 1-Pentanol, 2-Pentanol) und Hexanol (beispielsweise 1-Hexanol, 2-Hexanol, 2-Methyl-1-pentanol), besonders bevorzugt ist die monofunktionelle Starterverbindung (S-2) Methanol. Die Starterverbindungen (S-2) liegen vorzugsweise bei 100 °C, besonders bevorzugt bereits bei 70 °C in flüssiger Form vor.

Die mindestens eine Starterverbindung (S-1) wird in der mindestens einen monofunktionellen Starterverbindung (S-2) ("Costarter") dispergiert bzw. aufgelöst. Das Verhältnis zwischen Starterverbindungen (S-1) und monofunktionellen Starterverbindungen (S-2) kann in weiten Grenzen variiert werden. Das jeweils gewählte Verhältnis hängt zum Einen vom angestrebten Produktmix ab, zum Anderen muss natürlich eine ausreichende Rührbarkeit der Dispersion bzw. der Lösung der Starterverbindungen (S-1) in den monofunktionellen Starterverbindungen (S-2) gewährleistet sein. Beispielsweise haben sich Verhältnisse von 75 Gew.-% Saccharose und 25 Gew.-% Methanol als für das erfindungsgemäße Verfahren sehr gut geeignet herausgestellt

Der Dispersion bzw. Lösung der mindestens einen Starterverbindung (S-1) in der mindestens einen monofunktionellen Starterverbindung (S-2) wird in einer bevorzugten Ausführungsform des erfindungsgemäßen Verfahrens ein Katalysator zugesetzt. Beispielsweise können Doppelmetallcyanid-Verbindungen (DMC-Verbindungen) als Katalysatoren zugesetzt werden. DMC-Katalysatoren, wie zum Beispiel in US 5,470,813, US 6,696,383, EP-A 0 700 949, EP-A 0 743 093, EP-A 0 761 708, WO-A 97/40086, WO-A 98/16310 und WO-A 00/47649 beschrieben, besitzen eine hohe Aktivität bei Alkylenoxidadditionsreaktionen an Starterverbindungen mit Zerewitinoffaktiven Wasserstoffatomen und ermöglichen die Herstellung von Polyetherpolyolen bei sehr geringen Katalysatorkonzentrationen (50 ppm oder weniger), so dass die Abtrennung des DMC-Katalysators aus dem Polyetherpolyol vor dessen Verarbeitung zu Polyurethanen, z. B. Polyurethan-Schaumstoffen, nicht mehr erforderlich ist. Hierdurch wird die Wirtschaftlichkeit der technischen Polyetherpolyol-Produktion deutlich gesteigert. Alternativ kann das erfindungsgemäße Verfahren auch durch Lewis-Säuren wie beispielsweise Bortrifluorid-Etherat katalysiert werden. Solche Katalysatoren besitzen jedoch untergeordnete Bedeutung aufgrund ihrer Neigung zur Bildung von Nebenprodukten.

Bevorzugt werden im erfindungsgemäßen Verfahren basische Katalysatoren wie beispielsweise Alkalimetallhydride, Alkalimetallcarboxylate (beispielsweise von monofunktionellen Carbonsäuren), Alkalimetallhydroxide, Alkalimetallalkoxide (beispielsweise von monofunktionellen Alkoholen) oder Amine eingesetzt. Eine Übersicht über für das erfindungsgemäße Verfahren geeignete Amine ist von M. Ionescu et al. in "Advances in Urethanes Science and Technology", 1998, 14, S. 151-218 gegeben worden. Beispielsweise können N,N-Dimethylbenzylamin, Dimethylaminopropanol, N-Methyldiethanolamin, Trimethylamin, Triethylamin, N,N-Dimethylcyclohexylamin, N-Methylpyrrolidin, N,N,N',N'-Tetramethylethylendiamin, Diazabicyclo[2,2,2]-octan, 1,4-Dimethylpiperazin, N-Methylmorpholin, unsubstituiertes Imidazol und / oder alkylsubstituierte Imidazolderivate eingesetzt werden. Besonders bevorzugt werden im erfindungsgemäßen Verfahren als basische Katalysatoren Alkalimetallhydroxide (wie beispielsweise Natriumhydroxid, Kaliumhydroxid oder Cäsiumhydroxid), Alkalimetallalkoxide monofunktioneller Alkohole (wie beispielsweise Kaliumalkoholate monofunktioneller Alkohole, besonders bevorzugt Kaliummethanolat), Imidazol oder alkylsubstituierte Imidazolderivate (wie beispielsweise N-Methylimidazol) eingesetzt. Die Alkalimetallhydroxide können als Feststoff oder als hochkonzentrierte wässrige Lösungen verwendet werden.

Die basischen Katalysatoren werden im Allgemeinen in auf die Endproduktmenge (d.h. auf die Summe der resultierenden Polyetherpolyole A und B bezogene Mengen von 0,004 bis 0,8 Gew.-%, bevorzugt 0,004 bis 0,15 Gew.-% eingesetzt. Bei der Verwendung von Katalysatoren auf Alkalimetallhydroxidbasis oder bei Verwendung von N-Methyldiethanolamin ist zu beachten, dass während der Alkylenoxidadditionsreaktion geringe Mengen dihydroxyfunktioneller Polyethermoleküle entstehen können, welche jedoch bei den üblicherweise eingesetzten Katalysatormengen keinen signifikanten Einfluss auf die Endproduktfunktionalität besitzen. Werden als monofunktionelle Starterverbindungen (S-2) mindestens ein monofunktionelles Amin und/oder als Starterverbindungen (S-1) mindestens ein Polyamin oder Isomere bzw. Isomerengemische des Toluylendiamins eingesetzt, so wird in einer bevorzugten Ausführungsform der Erfindung in Schritt (ii) kein Katalysator eingesetzt.

Zur der aus Schritt (i) resultierenden Lösung bzw. Dispersion enthaltend mindestens eine Starterverbindung (S-1) und mindestens eine monofunktionelle Starterverbindung (S-2) wird mindestens ein Alkylenoxid, vorzugsweise unter Inertgasatmosphäre, kontinuierlich dosiert (Schritt (ii), "Alkoxylierung"). Das Alkylenoxid ist vorzugsweise mindestens eines ausgewählt aus der Gruppe bestehend aus Ethylenoxid, Propylenoxid, 1,2-Butylenoxid, 2,3-Butylenoxid und Styroloxid. Besonders bevorzugt sind Ethylenoxid, Propylenoxid oder eine Mischung von Propylenoxid und Ethylenoxid. Die Alkylenoxide können dem Reaktionsgemisch einzeln, im Gemisch oder nacheinander zugeführt werden. Werden die Alkylenoxide nacheinander dosiert, so enthalten die hergestellten Polyetherpolyole Polyetherketten mit Blockstrukturen. Produkte mit Ethylenoxidendblöcken sind beispielsweise durch einen erhöhten Anteil an primären Endgruppen gekennzeichnet, welche dem Polyetherpolyol eine erhöhte Reaktivität gegenüber Isocyanaten verleihen. Die Auswahl des jeweils verwendeten Alkylenoxids und deren Anordnung innerhalb der Polyetherketten werden im Hinblick auf die gewünschten Eigenschaften der angestrebten Polyetherpolyole A und B getroffen.

Vorzugsweise erfolgt die Alkoxylierung bei einer Temperatur von 70 bis 170°C, besonders bevorzugt bei einer Temperatur von 100 bis 130°C. Die Temperatur kann während der Alkylenoxiddosierphase (Schritt (ii)) innerhalb der beschriebenen Grenzen variiert werden: Um eine optimale Abstimmung zwischen hohem Alkylenoxidumsatz und geringer Nebenproduktbildung bei Verwendung empfindlicher Starterverbindungen (wie beispielsweise Saccharose) zu erreichen, kann zunächst bei niedrigen Reaktionstemperaturen (beispielsweise bei 70 bis 110°C) alkoxyliert werden, und erst bei hinreichendem Starterumsatz (d.h. sobald mindestens 50 Gew.-% der eingesetzten Starterverbindungen an mindestens einem Zerewitinoff-aktiven Wasserstoffatom mit Alkylenoxid reagiert haben) zu höheren Reaktionstemperaturen (beispielsweise auf 110 bis 130 °C) übergegangen werden. Nachreaktionen können ebenfalls bei höheren Temperaturen (d.h. nach Anhebung der Temperatur auf 100 bis 170 °C, bevorzugt 100 bis 150 °C) durchgeführt werden. Die Temperatur der exothermen Alkylenoxidadditionsreaktion wird durch Kühlung auf dem gewünschten Niveau gehalten. Gemäß dem Stand der Technik zur Auslegung von Polymerisationsreaktoren für exotherme Reaktionen (z. B. Ullmann's Encyclopedia of Industrial Chemistry, Band B4, Seite 167ff, 5. Ausgabe, 1992) erfolgt eine solche Kühlung im Allgemeinen über die Reaktorwand (z. B. Doppelmantel, Halbrohrschlange) sowie mittels weiterer intern im Reaktor und/oder extern im Umpumpkreislauf angeordneter Wärmetauscherflächen, z.B. an Kühlschlangen, Kühlkerzen, Platten- Rohrbündel- oder Mischerwärmetauschern. Diese sollten vorteilhafterweise so ausgelegt sein, dass auch zu Beginn der Dosierphase, d. h. bei kleinem Füllstand, und in Gegenwart eines heterogenen Reaktorinhalts (beispielsweise bei Vorliegen von Feststoffdispersionen) effektiv gekühlt werden kann.

Generell ist in allen Reaktionsphasen durch Auslegung und Einsatz handelsüblicher Rührorgane für eine gute Durchmischung des Reaktorinhaltes zu sorgen, wobei hier insbesondere ein- oder mehrstufig angeordnete Rührer oder großflächig über die Füllhöhe wirkende Rührertypen geeignet sind (siehe z.B. Handbuch Apparate; Vulkan-Verlag Essen, 1. Aufl. (1990), S.188 - 208). Besonders technisch relevant ist hierbei eine im Mittel über den gesamten Reaktorinhalt eingetragene volumenspezifische Mischleistung im Allgemeinen im Bereich von 0,2 bis 5 W/1 mit entsprechend höheren volumenspezifischen lokalen Leistungseinträgen im Bereich der Rührorgane selbst und ggf. bei niedrigeren Füllständen. Um eine optimale Rührwirkung zu erzielen, können im Reaktor gemäß allgemeinem Stand der Technik eine Kombinationen aus Stromstörern (z. B Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Energieeintrag zu gewährleisten. Beispielsweise kann es vorteilhaft sein, feststoffhaltige Dispersionen, die zu Reaktionsbeginn bei der Verwendung von Saccharose vorliegen können, besonders intensiv zu durchmischen. Außerdem ist beim Einsatz fester Starter durch die Wahl des Rühraggregates sicherzustellen, dass eine ausreichende Dispergierung des Feststoffes im Reaktionsgemisch gewährleistet ist. Bevorzugt werden hier bodengängige Rührstufen sowie besonders zur Suspendierung geeignete Rührorgane eingesetzt. Ferner sollte die Rührergeometrie zur Minderung des Aufschäumens von Reaktionsprodukten, wie beispielsweise nach Ende der Dosier- und Nachreaktionsphase bei der Abtrennung von Restepoxiden im Vakuum, beitragen. Hierbei haben sich Rührorgane als geeignet herausgestellt, die eine kontinuierliche Durchmischung der Flüssigkeitsoberfläche erzielen. Je nach Anforderung weist die Rührwelle ein Bodenlager und ggf. weitere Stützlager im Behälter auf. Der Antrieb der Rührerwelle kann dabei von oben oder unten erfolgen (mit zentrischer oder exzentrischer Anordnung der Welle). Alternativ ist es natürlich auch möglich die notwendige Durchmischung ausschließlich über einen über einen Wärmetauscher geführten Umpumpkreislauf zu erzielen oder diesen zusätzlich zum Rühraggregat als weitere Mischkomponente zu betreiben, wobei der Reaktorinhalt nach Bedarf (typischerweise 1 - 50 mal pro Stunde) umgepumpt wird.

Die kontinuierliche Dosierung des mindestens einen Alkylenoxids erfolgt so, dass die sicherheitstechnischen Druckgrenzen nicht überschritten werden. Diese richten sich naturgemäß nach den im Einzelfall vorliegenden apparativen Gegebenheiten, wobei der Prozess im Allgemeinen in einem Druckbereich von 1 mbar bis 10 bar, besonders bevorzugt von 1 mbar bis 4 bar ausgeführt wird. Insbesondere bei der Dosierung von ethylenoxidhaltigen Alkylenoxidgemischen oder reinem Ethylenoxid ist vorteilhafterweise darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden.

Das mindestens eine Alkylenoxid kann dem Reaktor auf unterschiedliche Weise zugeführt werden: Möglich ist eine Dosierung in die Gasphase oder direkt in die Flüssigphase, z.B. über ein Tauchrohr oder einen in der Nähe des Reaktorbodens in einer gut durchmischten Zone befindlichen Verteilerring. Wird ein Alkylenoxidgemisch dosiert, können die jeweiligen Alkylenoxide dem Reaktor separat oder als Mischung zugeführt werden. Eine Vorvermischung der Alkylenoxide kann beispielsweise durch ein in der gemeinsamen Dosierstrecke befindliches Mischaggregat erreicht werden ("inline-blending"). Es hat sich auch bewährt Alkylenoxide pumpendruckseitig in den Umpumpkreislauf einzeln oder vorgemischt zu dosieren. Für die gute Durchmischung mit dem Reaktionsmedium ist es dann von Vorteil ein hochscherendes Mischaggregat in den Alkylenoxid- / Reaktionsmediumstrom zu integrieren. Nach Ende der Alkylenoxiddosierung schließt sich vorzugsweise eine Nachreaktion an, deren Ende erreicht ist, sobald kein weiterer Druckabfall im Reaktionskessel mehr beobachtet wird. Gegebenenfalls können Restgehalte an Alkylenoxid danach auch noch durch einen Vakuum-, Inertgas- oder Dampfstrippschritt entfernt werden. Es ist auch möglich, Restgehalte an Alkylenoxid erst bei Durchführung von Schritt iv), d.h. der destillativen Abtrennung des monofunktionellen Polyetherpolyols B, zu entfernen. Die OH-Zahl des aus Schritt ii) resultierenden Rohgemisches (enthaltend Polyetherpolyol A und monofunktionelles Polyetherpolyol B) beträgt im Allgemeinen von 150 bis 1200 mg KOH / g, bevorzugt von 200 bis 1200 mg KOH / g und besonders bevorzugt von 270 bis 1200 mg KOH / g.

Generell sind die unterschiedlichsten Reaktortypen für die Durchführung des erfindungsgemäßen Verfahrens geeignet. Im Allgemeinen werden zylinderförmige Behälter eingesetzt, welche ein Höhen- zu Durchmesserverhältnis von 1:1 bis 10:1 besitzen. Als Reaktorböden kommen beispielsweise Kugel-, Klöpper-, Flach,- oder Konusböden in Frage.

Das aus Schritt ii) resultierende Rohgemisch kann optional Aufarbeitungsschritten unterzogen werden, um etwaige Katalysatorspuren zu entfernen (Schritt iii)). Im Falle von mit Aminen oder hochaktiven DMC-Verbindungen katalysierten Alkylenoxidadditionsreaktionen sind solche Nachbehandlungsschritte im Allgemeinen nicht erforderlich. Die optionale Entfernung des Katalysators aus dem aus Schritt ii) resultierenden Rohgemisches kann auf verschiedene Weise erfolgen: Beispielsweise kann der basische Katalysator mit verdünnten Mineralsäuren wie Schwefelsäure oder Phosphorsäure neutralisiert werden. Die bei der Neutralisation entstehenden Salze werden abgetrennt, beispielsweise durch Filtration. Ausnahmen bilden die in EP-A 2028211 und WO-A 2009106244 beschriebenen Polyetherpolyolherstellverfahren. Alternativ kann die Neutralisation mit Hydroxycarbonsäuren (wie beispielsweise Milchsäure, wie in WO-A 9820061 und US-A 2004167316 beschrieben) erfolgen. Ebenso geeignet zur Neutralisation sind Carbonsäuren wie beispielsweise Ameisensäure (vgl. US 4,521,548). Die nach Neutralisation mit Carbonsäuren (wie beispielsweise Hydroxycarbonsäuren oder Ameisensäure) entstehenden Metallcarboxylate sind in den Polyetherpolyolen klar löslich, so dass die Abtrennung der Salze hier entfallen kann. Ebenfalls möglich ist zur Entfernung des Katalysators der Einsatz von sauren Kationenaustauschern, wie beispielsweise in DE-A 100 24 313 beschrieben. Des Weiteren können die Katalysatoren mittels Adsorbentien wie beispielsweise Schichtsilikaten (Bentonit, Attapulgit), Diatomeenerde oder auch synthetischen Magnesiumsilikaten (wie AMBOSOL^{®} oder BriteSorb^{®}) abgetrennt werden. Solche Aufreinigungsverfahren sind beschrieben in RO 118433, US 4,507,475, EP-A 0693513 und EP-A 1751213. Phasentrennverfahren sind prinzipiell ebenfalls möglich, jedoch sind im Allgemeinen die Wasserlöslichkeiten der hochfunktionellen und der monofunktionellen Bestandteile des Reaktionsgemisches für eine effektive Durchführung von Phasentrennverfahren zu hoch. Phasentrennverfahren sind beispielsweise beschrieben in WO-A 0114456, JP-A 6-157743, WO-A 9620972 und US-A 3823145.

In Schritt iv) des erfindungsgemäßen Verfahrens wird aus dem nach Alkoxylierung (Schritt ii) und ggf. nach Entfernung des Katalysators (Schritt iii) resultierenden Polyetherpolyolgemisch mittels Destillation unter vermindertem Druck oder mittels Strippen mit Inertgas oder Wasser das monofunktionelle Polyetherpolyol B entfernt, wobei der verbleibende Destillationsrückstand das Polyetherpolyol A und bis zu 15 Gew.-% an monofunktionellem Polyetherpolyol B enthält. Die Abtrennung des monofunktionellen Polyetherpolyols B kann auch unter Zuhilfenahme von Dünnschichtverdampfern, Fallfilmverdampfern oder Schlangenrohrverdampfern bewerkstelligt werden, wobei auch hier der Abtrennprozess durch Einleitung von Inertgasströmen unterstützt werden kann. Solche Verfahren und Apparate sind beispielsweise beschrieben in "Perry's Chemical Engineers' Handbook"; 6. internationale Ausgabe.; 1984; Herausgeber: R. H. Perry, D. W. Green, J. O. Maloney; Mc Graw-Hill Book Company auf S. 11-34 bzw. in Kapitel 18, außerdem in DE 2755089 und WO-A 2010003734. Die Destillations- bzw. Stripptemperaturen können innerhalb eines weiten Bereiches gewählt werden. Sie richten sich an der thermischen Stabilität des Rohpolyethergemisches aus, insbesondere werden sie bestimmt durch die thermische Stabilität der Polyetherpolyols A. Im Allgemeinen liegen sie zwischen 100 und 200 °C. Werden Kurzwegverdampferapparaturen eingesetzt, können auch höhere Temperaturen gewählt werden, da hierbei generell kürzere Verweilzeiten der Produkte auf den beheizten Flächen realisierbar sind.

Alternativ ist es auch möglich, zuerst die Trennung gemäß Schritt iv) vorzunehmen, und anschließend den Katalysator aus monofunktionellem Polyetherpolyolen B bzw. dem Rückstand enthaltend Polyetherpolyol A und bis zu 15 Gew.-% monofunktionellem Polyetherpolyol B zu entfernen.

Der gemäß Schritt iv) verbleibende Rückstand enthält bevorzugt 85 bis 100 Gew.-%, besonders bevorzugt 95 bis 100 Gew.-% Polyetherpolyol A und bevorzugt 0 bis 15 Gew.-%, besonders bevorzugt 0 bis 5 Gew.-% (jeweils bezogen auf die Summe der Gew.-% des Polyetherpolyols A und des monofunktionellen Polyetherpolyols B) monofunktionelles Polyetherpolyol B.

Die nach dem erfindungsgemäßen Verfahren erhältlichen getrennten Bestandteile aus Polyetherpolyol A und monofunktionellem Polyetherpolyol B können jeweils weiteren Alkylenoxidadditionsreaktionen unterzogen werden, d.h. mit einem oder mehreren der oben genannten Alkylenoxide zur Reaktion gebracht werden. Im Allgemeinen kommen hierzu die oben genannten Katalysatoren in Betracht. Es kann bei den weiteren Alkylenoxidadditionsreaktionen der gleiche Katalysator wie in Schritt ii) oder ein von dem in Schritt ii) eingesetzten Katalysator verschiedener Katalysator eingesetzt werden. Die Zugabe von Katalysator erfolgt im Allgemeinen dann, wenn gemäß Schritt iii) der Katalysator vor der Auftrennung des Polyetherpolyolgemisches (Schritt iv) bereits abgetrennt wurde. Die Zugabe von Katalysator zu Polyol B erfolgt vorzugsweise auch dann, wenn in Schritt ii) ein schwerflüchtiger Katalysator eingesetzt wurde, der für eine weitere Alkylenoxidaddition an Polyol B nicht zur Verfügung steht. Ein Katalysatorwechsel ist bei den weiteren Alkylenoxidadditionsreaktionen möglich, beispielsweise von basischen Katalysatortypen hin zu sauren Katalysatortypen oder hin zu DMC-Katalysatoren. Wird das erfindungsgemäße Verfahren ohne Abtrennung des Katalysators gemäß Schritt iii) durchgeführt, so können die in Polyetherpolyol A und monofunktionellem Polyetherpolyol B verbliebenen Katalysatormengen auch direkt in den weiteren Alkylenoxidadditionsreaktion als Katalysator dienen. Gegebenenfalls sind die in Polyetherpolyol A und / oder Polyol B verbliebenen Katalysatormengen vor der Durchführung weiterer Alkylenoxidadditionsreaktionen aufzustocken. Eine bevorzugte Ausführungsform der Erfindung ist somit ein Koppelverfahren zur Herstellung eines zweiten Polyetherpolyols C und eines zweiten, monofunktionellen Polyetherpolyols D, dadurch gekennzeichnet, dass
(i) eine Lösung bzw. Dispersion einer oder mehrerer Starterverbindungen (S-1), die jeweils mindestens vier Zerewitinoff-aktive Wasserstoffatome aufweisen, in einer oder mehreren monofunktionellen Starterverbindungen (S-2) hergestellt wird, wobei das Gewichtsverhältnis von S-1 zu S-2 20 : 80 bis 85 : 15, bevorzugt 50 : 50 bis 85 : 15, besonders bevorzugt 60 : 40 bis 85 : 15 beträgt,
(ii) die aus Schritt (i) resultierende Lösung bzw. Dispersion mit einem oder mehreren Alkylenoxiden unter Zusatz eines Katalysators ausgewählt aus der Gruppe bestehend aus den basischen Katalysatoren, zur Reaktion gebracht wird, wobei das Umsetzungsprodukt aus der mindestens einen Starterverbindungen (S-1) mit einem oder mehreren Alkylenoxiden das Polyetherpolyol A ist, und wobei das Umsetzungsprodukt aus der mindestens einen monofunktionellen Starterverbindungen (S-2) mit einem oder mehreren Alkylenoxiden das monofunktionelle Polyetherpolyol B ist,
(iii) das resultierende Gemisch nicht vom Katalysator befreit wird,
(iv) das monofunktionelle Polyetherpolyol B durch Destillation unter vermindertem Druck oder durch Strippen mit Inertgas oder Wasser abgetrennt wird, wobei der verbleibende Rückstand Polyetherpolyol A und bis zu 15 Gew.% (jeweils bezogen auf die Summe der Gew.-% des Polyetherpolyols A und des monofunktionellen Polyetherpolyols B) an monofunktionellem Polyetherpolyol B enthält, und
(v)
   (1) der verbleibende Rückstand enthaltend Polyetherpolyol A und bis zu 15 Gew.-% (jeweils bezogen auf die Summe der Gew.-% des Polyetherpolyols A und des monofunktionellen Polyetherpolyols B) an monofunktionellem Polyetherpolyol B, gegebenenfalls nach Zugabe von weiterem Katalysator mit einem oder mehreren Alkylenoxiden zur Reaktion gebracht wird, wobei das Polyetherpolyol C erhalten wird und/oder
   (2) das monofunktionelle Polyetherpolyol B, gegebenenfalls nach Zugabe von weiterem Katalysator, mit einem oder mehreren Alkylenoxiden zur Reaktion gebracht wird, wobei das Polyetherpolyol D erhalten wird.

Dem Polyetherpolyol A und/oder dem monofunktionellen Polyetherpolyol B sowie dem Polyetherpolyol C und dem Polyetherpolyol D können Antioxidanzmittel (z. B. auf Basis von Phenolderivaten und / oder auf Basis von Aminen) zugesetzt werden. Solche Antioxidanzmittel werden im Allgemeinen erst nach Abtrennung basischer, insbesondere erst nach Abtrennung alkalimetallhaltiger Katalysatorspuren zugesetzt, da auf diese Weise Verfärbungen des jeweiligen Polyetherpolyols vermieden werden können.

Die nach dem erfindungsgemäßen Verfahren erhältlichen Polyetherpolyole A, B, C und/oder D können als Ausgangskomponenten für die Herstellung von massiven oder geschäumten Polyurethanwerkstoffen sowie von Polyurethanelastomeren eingesetzt werden. Die Polyurethanwerkstoffe und -elastomere können auch Isocyanurat-, Allophanat- und Biuretstruktureinheiten enthalten. Ebenfalls möglich ist die Herstellung sogenannter Isocyanat-Prepolymere, bei deren Herstellung mindestens ein (Poly-)Isocyanat und mindestens ein Polyetherpolyol A, B, C und/oder D eingesetzt werden, wobei das molare Verhältnis von Isocyanatgruppen zu Hydroxygruppen größer als 1 beträgt, so dass die resultierenden Prepolymere Isocyanatgruppen enthalten. Diese Isocyanatgruppen der Prepolymere können in einem oder mehreren Schritten mit Verbindungen enthaltend Zerewitinoff-aktive Wasserstoffatome umgesetzt werden zur Herstellung des eigentlichen Endproduktes wie massive oder geschäumte Polyurethanwerkstoffe sowie von Polyurethanelastomeren.

Zur Herstellung von massiven oder geschäumten Polyurethanwerkstoffen sowie von Polyurethanelastomeren werden die erfindungsgemäßen Polyetherpolyole A, B, C und/oder D gegebenenfalls mit weiteren isocyanatreaktiven Komponenten gemischt und mit organischen Polyisocyanaten, gegebenenfalls in Gegenwart von Treibmitteln in Gegenwart von Katalysatoren, gegebenenfalls in Gegenwart anderer Zusatzstoffe wie z. B. Zellstabilisatoren, zur Reaktion gebracht.

### Beispiele

### Ambosol® :

Ausgefälltes, kolloidales, synthetisch hergestelltes Magnesiumsilikat (Zusammensetzung: 2,7 SiO₂, MgO, 1,5 H₂O)

### Katalysator:

25 Gew.-%ige Lösung von Kaliummethoxid (Kaliummethanolat) in Methanol

### Methoden:

Die Bestimmung der OH-Zahlen erfolgte gemäß der Vorschrift der DIN 53240.

Die Molmassenverteilung wurde mittels Größenausschlusschromatographie (SEC) ermittelt. Verwendet wurde das Gerät Agilent 1100 Series der Fa. Agilent. Angegeben wird die Polydispersität (PD) für die Molekulargewichtsverteilung M_{w}/Mₙ, wobei M_{w} für die gewichtsgemittelte Molmasse und Mₙ für die zahlengemittelte Molmasse stehen. Weitere Angaben:
- Säulenkombination: 1 Vorsäule PSS, 5 µl, 8x50mm; 2 PSS SVD, 5 µl, 100 A°, 8x300mm; 2 PSS SVD, 5 µl, 1000 A°, 8x300mm, PSS ist der Hersteller der Säulen (Polymer Standard Solutions, Mainz)
- Auswertesoftware: WIN GPC der Fa. PSS
- Lösungsmittel: THF (Merck LiChrosolv)
- Flussrate: 1 ml / min
- Detektortyp: RI-Detektor (Brechungsindex), Shodex RI 74
- Verwendete Kalibrationsstandards: Kalibrierstandard der Fa PSS auf Basis Polystyrol.

### Beispiel 1:

In einen 2 1 Laborautoklaven wurden unter Stickstoffatmosphäre 52,46 g Methanol, 140,9 g Saccharose und 10,3 g einer 25 %igen Lösung von Kaliummethoxid in Methanol gegeben. Restsauerstoff wurde nach Schließen des Befüllstutzens durch jeweils dreimaliges Befüllen der Apparatur mit 3,0 bar Stickstoff und anschließendes Ablassen des Überdrucks auf Atmosphärendruck entfernt. Der Inhalt des Autoklaven wurde unter Rühren (800 U/min.) auf 110 °C erwärmt. Man dosierte 486 g Propylenoxid bei einer Rührerdrehzahl von 800 U/min. über einen Zeitraum von 3,0 h in den Autoklaven. Die Propylenoxiddosierung wurde bei einem Druck von 5,0 bar gestartet, und das während der Dosierphase erreichte Druckmaximum betrug 6,0 bar. Nach Ende der Propylenoxiddosierung schloss sich eine Nachreaktionszeit von 8 h an. Nach einer Ausheizphase von 30 min. Dauer im Vakuum (10 mbar) wurde das resultierende Gemisch unter Rühren auf 80 °C abgekühlt und mit 20 g Ambosol^{®} als Adsorptionsmittel versetzt. Es wurde 2 h gerührt und danach über ein Tiefenfilter (T 750) filtriert, um das Adsorptionsmittel abzutrennen.

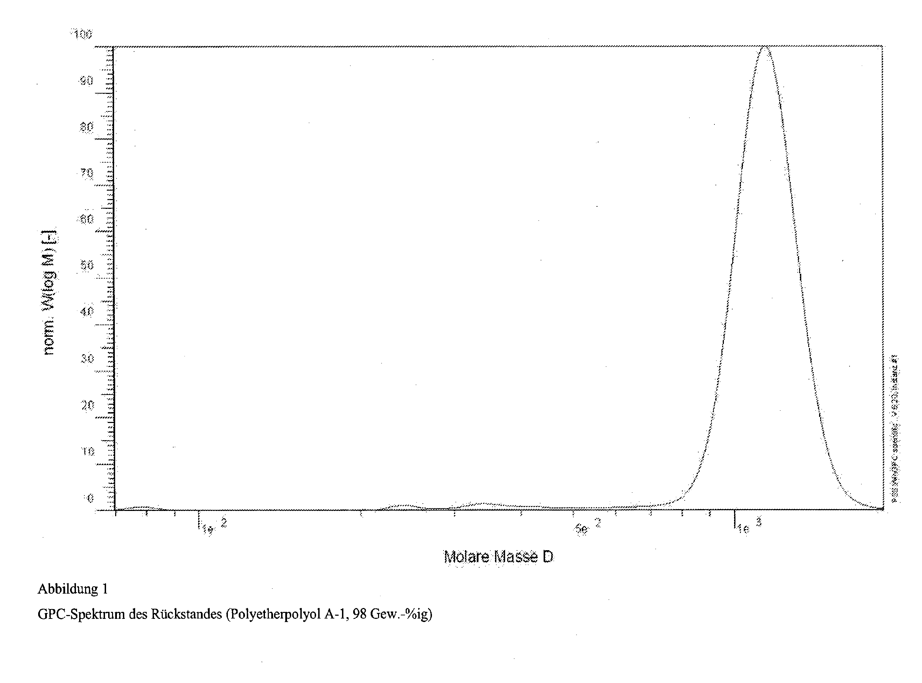

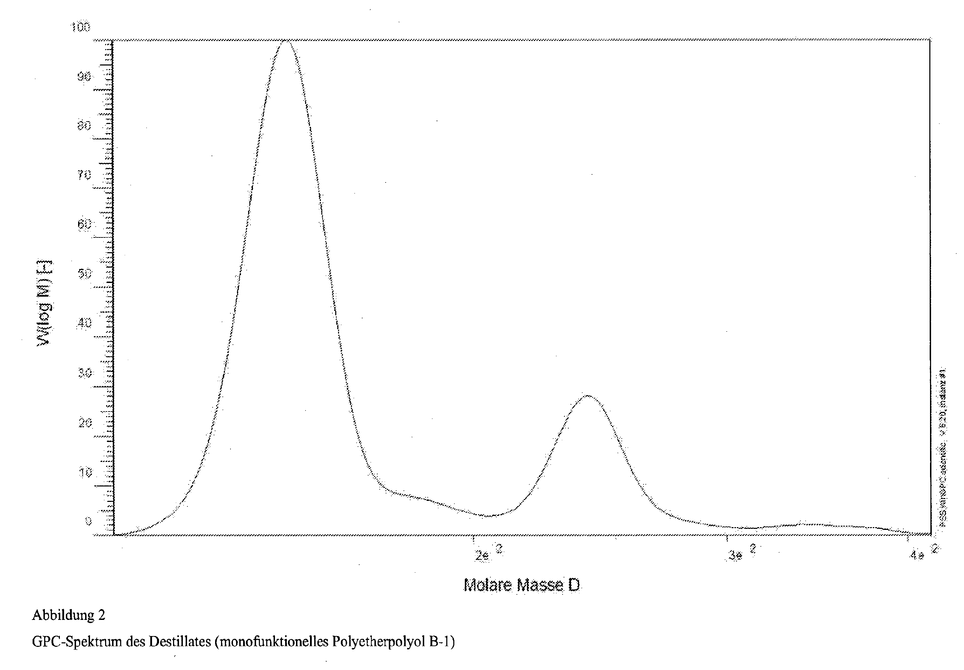

251,6 g des erhaltenen Filtrates wurden zunächst bei 120 °C und 1 mbar 9,5 h gerührt, wobei mittels Destillation 42,87 g Destillat (monofunktionelles Polyetherpolyol B-1, basierend auf Methanol als Starterverbindung) abgetrennt wurden. Danach wurde noch 10 h bei 150 °C mit Stickstoff gestrippt, d.h. bei angelegtem Vakuum (erzeugt mittels einer Ölpumpe) wurde unter Rühren ein leichter Stickstoffstrom mittels Glasrohr in die Füssigphase eingeleitet. Hierbei konnten weitere 2,6 g flüchtiges Material (monofunktionelles Polyetherpolyol B-1) in Vorlage und Kühlfalle aufgefangen werden. Die beiden Destillat-Fraktionen wurden vereinigt und mittels GPC analysiert (Abbildung 2, monofunktionelles Polyetherpolyol B-1). Der Rückstand wies eine Polydispersität (PD) von 1,09 auf, seine OH-Zahl betrug 370 mg KOH / g. Aus den Integralen der Signale des GPC-Spektrums des Rückstands (Abbildung 1) wurde der Anteil des Polyetherpolyols A-1 (basierend auf Saccharose als Starterverbindung) zu 98 Gew.-% in diesem Rückstand ermittelt. Der Auswertung des GPC-Spektrums wurde hierbei zugrunde gelegt, dass die Signalintensität proportional zur Masse der jeweiligen Spezies ist. Des Weiteren wurden Signale unterhalb 500 Da dem monofunktionellen Polyetherpolyol B-1 zugerechnet, und Signale oberhalb von 500 Da wurden dem Polyetherpolyol A-1 zugerechnet.

## Patentansprüche

1. Verfahren zur Herstellung eines ersten Polyetherpolyols A und eines zweiten, monofunktionellen Polyetherpolyols B, **dadurch gekennzeichnet, dass**
(i) eine Lösung bzw. Dispersion einer oder mehrerer Starterverbindungen (S-1), die jeweils mindestens vier Zerewitinoff-aktive Wasserstoffatome aufweisen, in einer oder mehreren monofunktionellen Starterverbindungen (S-2) hergestellt wird, wobei das Gewichtsverhältnis von S-1 zu S-2 20 : 80 bis 85 : 15 beträgt,
(ii) die aus Schritt (i) resultierende Lösung bzw. Dispersion mit einem oder mehreren Alkylenoxiden, gegebenenfalls nach Zusatz eines Katalysators, zur Reaktion gebracht wird, wobei das Umsetzungsprodukt aus der mindestens einen Starterverbindungen (S-1) mit einem oder mehreren Alkylenoxiden das Polyetherpolyol A ist, und wobei das Umsetzungsprodukt aus der mindestens einen monofunktionellen Starterverbindungen (S-2) mit einem oder mehreren Alkylenoxiden das monofunktionelle Polyetherpolyol B ist,
(iii) gegebenenfalls das resultierende Gemisch vom Katalysator befreit wird, und
(iv) das monofunktionelle Polyetherpolyol B durch Destillation unter vermindertem Druck oder durch Strippen mit Inertgas oder Wasser abgetrennt wird, wobei der verbleibende Rückstand Polyetherpolyol A und bis zu 15 Gew.-% (jeweils bezogen auf die Summe der Gew.-% des Polyetherpolyols A und des monofunktionellen Polyetherpolyols B) an monofunktionellem Polyetherpolyol B enthält.

2. Verfahren gemäß Anspruch 1, wobei in Schritt (i) das Gewichtsverhältnis von S-1 zu S-2 60 : 40 bis 85 : 15 beträgt.

3. Verfahren gemäß Anspruch 1, wobei als Starterverbindungen (S-1) mindestens eine Verbindung ausgewählt aus der Gruppe bestehend aus Mono-, Oligo- und Polysacchariden, Pentaerythrit, Dipentaerythrit, Tripentaerythrit, , Sorbit, cyclischen Polyolen, Polyaminen und Isomeren bzw. Isomerengemischen des Toluylendiamins und als monofunktionelle Starterverbindungen (S-2) mindestens eine Verbindung ausgewählt aus der Gruppe bestehend aus monofunktionellen Alkoholen, kurzkettigen monofunktionellen Monoalkylethern von Glykolen, höheren monofunktionellen Fettalkoholen, monofunktionellen Aminen und monofunktionellen Alkanolaminen eingesetzt werden.

4. Verfahren gemäß Anspruch 1, wobei als Starterverbindungen (S-1) Saccharose und/oder Pentaerythrit und als monofunktionelle Starterverbindungen (S-2) mindestens eine Verbindung ausgewählt aus der Gruppe bestehend aus Methanol, Ethanol, Propanol, Butanol, Pentanol und Hexanol eingesetzt werden.

5. Verfahren gemäß einem der Ansprüche 1 bis 4, wobei in Schritt (ii) ein Katalysator ausgewählt aus der Gruppe bestehend aus Doppelmetallcyanid-Verbindungen (DMC-Verbindungen), Alkalimetallhydriden, Alkalimetallcarboxylaten, Alkalimetallhydroxiden, Alkalimetallalkoxiden und Aminen eingesetzt wird.

6. Verfahren gemäß Anspruch 5, wobei in Schritt (ii) ein Katalysator ausgewählt aus der Gruppe bestehend aus Alkalimetallhydroxid, Alkalimetallalkoxid eines monofunktionellen Alkohols, Imidazol und alkylsubstituierts Imidazolderivat eingesetzt wird.

7. Verfahren gemäß Anspruch 5, wobei in Schritt (ii) Kaliummethanolat als Katalysator eingesetzt wird.

8. Verfahren gemäß Anspruch 1, wobei der in Schritt iv) verbleibende Destillationsrückstand 95 bis 100 Gew.-% Polyetherpolyol A und 0 bis 5 Gew.-% (jeweils bezogen auf die Summe der Gew.-% des Polyetherpolyols A und des monofunktionellen Polyetherpolyols B) monofunktionelles Polyetherpolyol B enthält.

9. Verfahren gemäß einem der Ansprüche 1 bis 7, wobei
(i) eine Lösung bzw. Dispersion einer oder mehrerer Starterverbindungen (S-1), die jeweils mindestens vier Zerewitinoff-aktive Wasserstoffatome aufweisen, in einer oder mehreren monofunktionellen Starterverbindungen (S-2) hergestellt wird, wobei das Gewichtsverhältnis von S-1 zu S-2 20 : 80 bis 85 : 15 beträgt,
(ii) die aus Schritt (i) resultierende Lösung bzw. Dispersion mit einem oder mehreren Alkylenoxiden unter Zusatz eines Katalysators ausgewählt aus der Gruppe bestehend aus den basischen Katalysatoren, zur Reaktion gebracht wird, wobei das Umsetzungsprodukt aus der mindestens einen Starterverbindungen (S-1) mit einem oder mehreren Alkylenoxide n das Polyetherpolyol A ist, und wobei das Umsetzungsprodukt aus der mindestens einen monofunktionellen Starterverbindungen (S-2) mit einem oder mehreren Alkylenoxiden das monofunktionelle Polyetherpolyol B ist,
(iii) das resultierende Gemisch nicht vom Katalysator befreit wird,
(iv) das monofunktionelle Polyetherpolyol B durch Destillation unter vermindertem Druck oder durch Strippen mit Inertgas oder Wasser abgetrennt wird, wobei der verbleibende Rückstand Polyetherpolyol A und bis zu 15 Gew.% (jeweils bezogen auf die Summe der Gew.-% des Polyetherpolyols A und des monofunktionellen Polyetherpolyols B) an monofunktionellem Polyetherpolyol B enthält, und
(v)
(1) der verbleibende Rückstand enthaltend Polyetherpolyol A und bis zu 15 Gew.-% (jeweils bezogen auf die Summe der Gew.-% des Polyetherpolyols A und des monofunktionellen Polyetherpolyols B) an monofunktionellem Polyetherpolyol B, gegebenenfalls nach Zugabe von weiterem Katalysator mit einem oder mehreren Alkylenoxiden zur Reaktion gebracht wird, wobei das Polyetherpolyol C erhalten wird und/oder
(2) das monofunktionelle Polyetherpolyol B, gegebenenfalls nach Zugabe von weiterem Katalysator mit einem oder mehreren Alkylenoxiden zur Reaktion gebracht wird, wobei das Polyetherpolyol D erhalten wird.

## Claims

1. Process for the preparation of a first polyether polyol A and of a second, monofunctional polyether polyol B, **characterised in that**
(i) a solution or dispersion of one or more starter compounds (S-1), each having at least four Zerewitinoff-active hydrogen atoms, in one or more monofunctional starter compounds (S-2) is prepared, the weight ratio of S-1 to S-2 being from 20:80 to 85:15,
(ii) the solution or dispersion resulting from step (i) is reacted with one or more alkylene oxides, optionally after addition of a catalyst, the reaction product of the at least one starter compound (S-1) with one or more alkylene oxides being the polyether polyol A, and the reaction product of the at least one monofunctional starter compound (S-2) with one or more alkylene oxides being the monofunctional polyether polyol B,
(iii) the resulting mixture is optionally freed of catalyst, and
(iv) the monofunctional polyether polyol B is separated off by distillation under reduced pressure or by stripping with inert gas or water, the residue that remains containing polyether polyol A and up to 15 wt.% (in each case based on the sum of the wt.% of the polyether polyol A and of the monofunctional polyether polyol B) of monofunctional polyether polyol B.

2. Process according to claim 1, wherein in step (i) the weight ratio of S-1 to S-2 is from 60:40 to 85:15.

3. Process according to claim 1, wherein there are used as starter compounds (S-1) at least one compound selected from the group consisting of mono-, oligo- and poly-saccharides, pentaerythritol, dipentaerythritol, tripentaerythritol, sorbitol, cyclic polyols, polyamines, and isomers or isomer mixtures of toluylenediamine, and as monofunctional starter compounds (S-2) at least one compound selected from the group consisting of monofunctional alcohols, short-chained monofunctional monoalkyl ethers of glycols, higher monofunctional fatty alcohols, monofunctional amines and monofunctional alkanolamines.

4. Process according to claim 1, wherein there are used as starter compounds (S-1) sucrose and/or pentaerythritol and as monofunctional starter compounds (S-2) at least one compound selected from the group consisting of methanol, ethanol, propanol, butanol, pentanol and hexanol.

5. Process according to any one of claims 1 to 4, wherein in step (ii) a catalyst selected from the group consisting of double metal cyanide compounds (DMC compounds), alkali metal hydrides, alkali metal carboxylates, alkali metal hydroxides, alkali metal alkoxides and amines is used.

6. Process according to claim 5, wherein in step (ii) a catalyst selected from the group consisting of alkali metal hydroxide, alkali metal alkoxide of a monofunctional alcohol, imidazole and alkylsubstituted imidazole derivative is used.

7. Process according to claim 5, wherein in step (ii) potassium methanolate is used as catalyst.

8. Process according to claim 1, wherein the distillation residue that remains in step iv) contains from 95 to 100 wt.% of polyether polyol A and from 0 to 5 wt.% (in each case based on the sum of the wt.% of the polyether polyol A and of the monofunctional polyether polyol B) of monofunctional polyether polyol B.

9. Process according to any one of claims 1 to 7, wherein
(i) a solution or dispersion of one or more starter compounds (S-1), each having at least four Zerewitinoff-active hydrogen atoms, in one or more monofunctional starter compounds (S-2) is prepared, the weight ratio of S-1 to S-2 being from 20:80 to 85:15,
(ii) the solution or dispersion resulting from step (i) is reacted with one or more alkylene oxides with the addition of a catalyst selected from the group consisting of the basic catalysts, the reaction product of the at least one starter compound (S-1) with one or more alkylene oxides being the polyether polyol A, and the reaction product of the at least one monofunctional starter compound (S-2) with one or more alkylene oxides being the monofunctional polyether polyol B,
(iii) the resulting mixture is not freed of catalyst,
(iv) the monofunctional polyether polyol B is separated off by distillation under reduced pressure or by stripping with inert gas or water, the residue that remains containing polyether polyol A and up to 15 wt.% (in each case based on the sum of the wt.% of the polyether polyol A and of the monofunctional polyether polyol B) of monofunctional polyether polyol B, and
(v)
(1) the residue that remains containing polyether polyol A and up to 15 wt.% (in each case based on the sum of the wt.% of the polyether polyol A and of the monofunctional polyether polyol B) of monofunctional polyether polyol B, optionally after addition of further catalyst, is reacted with one or more alkylene oxides, the polyether polyol C being obtained, and/or
(2) the monofunctional polyether polyol B, optionally after addition of further catalyst, is reacted with one or more alkylene oxides, the polyether polyol D being obtained.

## Revendications

1. Procédé pour la préparation d'un premier polyétherpolyol A et d'un deuxième polyétherpolyol monofonctionnel B, **caractérisé en ce que**
(i) on prépare une solution ou une dispersion d'un ou de plusieurs composés de départ (S-1), qui présentent à chaque fois au moins quatre atomes d'hydrogène actifs selon Zerewitinoff, dans un ou plusieurs composés de départ monofonctionnels (S-2), le rapport pondéral de S-1 à S-2 valant 20:80 à 85:15,
(ii) on fait réagir la solution ou la dispersion résultant de l'étape (i) avec un ou plusieurs oxydes d'alkylène, le cas échéant après addition d'un catalyseur, le produit de transformation dudit au moins un composé de départ (S-1) avec un ou plusieurs oxydes d'alkylène étant le polyétherpolyol A et le produit de transformation dudit au moins un composé de départ monofonctionnel (S-2) avec un ou plusieurs oxydes d'alkylène étant le polyétherpolyol monofonctionnel B,
(iii) on libère le cas échéant le mélange résultant du catalyseur et
(iv) on sépare le polyétherpolyol monofonctionnel B par distillation sous pression réduite ou par rectification avec un gaz inerte ou de l'eau, le résidu restant contenant du polyétherpolyol A et jusqu'à 15% en poids (à chaque fois par rapport à la somme des % en poids du polyétherpolyol A et du polyétherpolyol monofonctionnel B) de polyétherpolyol monofonctionnel B.

2. Procédé selon la revendication 1, où, dans l'étape (i), le rapport pondéral de S-1 à S-2 vaut 60:40 à 85:15.

3. Procédé selon la revendication 1, où on utilise, comme composés de départ (S-1), au moins un composé choisi dans le groupe constitué par les monosaccharides, les oligosaccharides et les polysaccharides, le pentaérythritol, le dipentaérythritol, le tripentaérythritol, le sorbitol, les polyols cycliques, les polyamines et les isomères ou mélanges d'isomères de la toluylènediamine et, comme composés de départ monofonctionnels (S-2), au moins un composé choisi dans le groupe constitué par les alcools monofonctionnels, les monoalkyléthers monofonctionnels à courte chaîne de glycols, les alcools gras monofonctionnels supérieurs, les amines monofonctionnelles et les alcanolamines monofonctionnelles.

4. Procédé selon la revendication 1, où on utilise, comme composés de départ (S-1), le saccharose et/ou le pentaérythritol et, comme composés de départ monofonctionnels (S-2), au moins un composé choisi dans le groupe constitué par le méthanol, l'éthanol, le propanol, le butanol, le pentanol et l'hexanol.

5. Procédé selon l'une quelconque des revendications 1 à 4, où, dans l'étape (ii), on utilise un catalyseur choisi dans le groupe constitué par les composés de type cyanure métallique double (composés DMC), les hydrures de métal alcalin, les carboxylates de métal alcalin, les hydroxydes de métal alcalin, les alcoxydes de métal alcalin et les amines.

6. Procédé selon la revendication 5, où, dans l'étape (ii), on utilise un catalyseur choisi dans le groupe constitué par un hydroxyde de métal alcalin, un alcoxyde de métal alcalin d'un alcool monofonctionnel, l'imidazole et un dérivé d'imidazole substitué par alkyle.

7. Procédé selon la revendication 5, où on utilise, dans l'étape (ii), du méthanolate de potassium comme catalyseur.

8. Procédé selon la revendication 1, où le résidu de distillation restant dans l'étape iv) contient 95 à 100% en poids de polyétherpolyol A et 0 à 5% en poids (à chaque fois par rapport à la somme des % en poids du polyétherpolyol A et du polyétherpolyol monofonctionnel B) de polyétherpolyol monofonctionnel B.

9. Procédé selon l'une quelconque des revendications 1 à 7, où
(i) on prépare une solution ou une dispersion d'un ou de plusieurs composés de départ (S-1), qui présentent à chaque fois au moins quatre atomes d'hydrogène actifs selon Zerewitinoff, dans un ou plusieurs composés de départ monofonctionnels (S-2), le rapport pondéral de S-1 à S-2 valant 20:80 à 85:15,
(ii) on fait réagir la solution ou la dispersion résultant de l'étape (i) avec un ou plusieurs oxydes d'alkylène en ajoutant un catalyseur choisi dans le groupe constitué par les catalyseurs basiques, le produit de transformation dudit au moins un composé de départ (S-1) avec un ou plusieurs oxydes d'alkylène étant le polyétherpolyol A et le produit de transformation dudit au moins un composé de départ monofonctionnel (S-2) avec un ou plusieurs oxydes d'alkylène étant le polyétherpolyol monofonctionnel B,
(iii) on ne libère pas le mélange résultant du catalyseur,
(iv) on sépare le polyétherpolyol monofonctionnel B par distillation sous pression réduite ou par rectification avec un gaz inerte ou de l'eau, le résidu restant contenant du polyétherpolyol A et jusqu'à 15% en poids (à chaque fois par rapport à la somme des % en poids du polyétherpolyol A et du polyétherpolyol monofonctionnel B) de polyétherpolyol monofonctionnel B, et
(v)
(1) on fait réagir le résidu restant contenant du polyétherpolyol A et jusqu'à 15 % en poids (à chaque fois par rapport à la somme des % en poids du polyétherpolyol A et du polyétherpolyol monofonctionnel B) de polyétherpolyol monofonctionnel B, le cas échéant après addition de catalyseur supplémentaire, avec un ou plusieurs oxydes d'alkylène, le polyétherpolyol C étant obtenu et/ou
(2) on fait réagir le polyétherpolyol monofonctionnel B, le cas échéant après addition de catalyseur supplémentaire, avec un ou plusieurs oxydes d'alkylène, le polyétherpolyol D étant obtenu.
